# EUROPÄISCHE PATENTSCHRIFT

(11) **EP 3 921 653 B1**
(45) Veröffentlichungstag und Bekanntmachung des Hinweises auf die Patenterteilung: **04.01.2023**
(21) Anmeldenummer: 20703952.0
(22) Anmeldetag: 30.01.2020
(51) Int. Cl.: G01N 35/02, G01N 1/28, G01N 23/2202, G01N 35/00, G01N 23/2204, G01N 23/223, G01N 23/2276, B23Q 7/14, G01N 33/205

(54) **VERFAHREN ZUR HANDHABUNG VON SCHMELZPROBEN IN EINEM STAHLWERKSLABOR SOWIE EIN STAHLWERKSLABOR**
METHOD FOR HANDLING MELT SAMPLES IN A STEELWORKS LABORATORY, AND STEELWORKS LABORATORY
PROCÉDÉ DE MANIPULATION D'ÉCHANTILLONS EN FUSION DANS UN LABORATOIRE SIDÉRURGIQUE ET LABORATOIRE SIDÉRURGIQUE

(30) Priorität: 07.02.2019 DE 102019103029
(43) Veröffentlichungstag der Anmeldung: 15.12.2021
(73) Patentinhaber: thyssenkrupp Industrial Solutions AG, 45143 Essen (DE); thyssenkrupp AG, 45143 Essen (DE)
(72) Erfinder: TEUTENBERG, Reinhard, 59423 Unna (DE); PETERS, Alexander, 59269 Beckum (DE)
(74) Vertreter: thyssenkrupp Intellectual Property GmbH
(86) Internationale Anmeldenummer: PCT/EP2020/052371
(87) Internationale Veröffentlichungsnummer: WO 2020/161003

(56) Entgegenhaltungen:
- DE-A1-102006 049 208
- DE-A1-102009 003 510
- Anonymous: "Rotary transfer machine - Wikipedia", , 11. Oktober 2018 (2018-10-11), XP055687078, Gefunden im Internet: URL:https://en.wikipedia.org/wiki/Rotary_t ransfer_machine [gefunden am 2020-04-20]

## Beschreibung

Die Erfindung betrifft eine Verfahren zur Handhabung von Schmelzproben in einem Stahlwerkslabor sowie ein Stahlwerkslabor, wobei die Schmelzprobe in wenigstens einer ersten Behandlungseinrichtung bearbeitet und/oder analysiert wird und anschließend über wenigstens einen ersten Transportweg zu wenigstens einer zweiten Behandlungseinrichtung transportiert wird, wo die Schmelzprobe einer weiteren Bearbeitung und/oder Analyse unterzogen wird.

In allen Phasen der Stahlerzeugung - von der Roheisengewinnung bis zum fertigen Produkt - werden modernste Fertigungs- und Messmethoden eingesetzt. Dabei ist die genaue Kenntnis der Stahlzusammensetzung ein wichtiger Aspekt, da von ihr neben der Art der Verarbeitung die Materialeigenschaften abhängen. Die Stahlzusammensetzung ergibt sich zu Beginn des Herstellungsprozesses aus den Ausgangsstoffen Eisenerz, Koks, den Reduktionsprozessen sowie der Hinzulegierung von Begleitelementen in der Sekundärmetallurgie. Konventionell wird die Zusammensetzung anhand von Stichproben aus der Roheisen- oder Stahlschmelze mittels Röntgen-Fluoreszenzanalytik (RFA), Verbrennungsanalyse und auch der Funkemissionsspektroskopie bestimmt. Diese Verfahren stellen hohe Ansprüche an die Probenpräparation.

Bei der konventionellen Prozesskontrolle in einem Stahlwerk wird eine aus einer Stahlschmelze mittels einer Tauchsonde gezogene Probe mittels Rohrposttransport zu einem Stahlwerkslabor transportiert. Je nach Bedarf ist die Probe vor der weiteren Verarbeitung zu kühlen und gelangt dann in eine Fräse um die Zunderschicht ganzflächig zu entfernen. Im Anschluss wird die vom Zunder befreite Probe einer Röntgenfluoreszenzanalyse unterzogen oder gegebenenfalls gleich mit der Funkenemissionsspektrometrie analysiert. Zusätzlich zur Funkenemissionsspektrometrie wird zur Bestimmung des Kohlenstoff- und Schwefelgehaltes bei einem Teil der Proben eine Verbrennungsanalyse durchgeführt. Hierfür wird üblicherweise ein Teil der beim Fräsen oder durch andere spanabhebende Verfahren gewonnenen Späne verwendet. Darüber hinaus können weitere Behandlungseinrichtungen vorgesehen sein.

Die Proben werden zwischen den einzelnen Behandlungseinrichtungen mit Hilfe eines Transportsystems (Förderbänder oder Schienensystem) transportiert. Auch ist es bekannt, dass die Proben mittels eines Roboters zu den nachgeschalteten Behandlungseinrichtungen überführt werden. An jeder Behandlungseinrichtung sind üblicherweise Übergabeeinheiten vorgesehen, die den Transfer der Probe vom Transportsystem in die Behandlungseinrichtung gewährleisten, wo die Proben positioniert und ggf. auch vermessen und behandelt werden.

Aus der EP 0 633 207 A1 ist ein Transportsystem zum Transport von Proben zu unterschiedlichen Behandlungseinrichtungen bekannt, wobei ein umlaufendes Transportband mit darauf umlaufenden Probenträgern zum Einsatz kommen. Die Proben sind dabei in einem Becher enthalten, der auf einem Probenträger angeordnet ist. Die Probenträger sind während des Transportes auf zwei Fördergurten des Transportbandes aufgesetzt. Vor einer Behandlungseinrichtung wird der Probenträger mit Hilfe einer Stoppvorrichtung angehalten und zusammen mit dem die Probe enthaltenden Becher mittels eines Handhabungsgeräts vom Transportband angehoben und in die Behandlungseinrichtung transferiert. Nach der Behandlung der Probe wird der Probenträger nebst dem die Probe enthaltenden Becher wieder auf das Transportband zurückgebracht, wobei es auch denkbar ist, dass die Probe in der Behandlungseinrichtung verbleibt und nur der leere Becher mit dem Probenträger auf das Transportband zurückgelangen. Aus der DE102009003510 befasst sich in Reinster Weise mit der Aufnahme der Probe. Es wird beschrieben, dass diese in einem Rohrpostbehälter transportiert werden kann. Die Problematik des Umspannens und erneutem Vermessen und Einstellen der Probe auf den folgenden Verfahrensschritt ist in der Beschreibung nicht beschrieben. DE102009003510 zeigt einen Roboter mit einem dreidimensional bewegbaren Greifarm, der die Probe greifen soll.

Der Erfindung liegt die Aufgabe zugrunde, die Handhabung von Schmelzproben bei der Stahlherstellung zu vereinfachen und die Zeitdauer für die Probenpräparation, das Probenhandling und die chemische Analyse zu verkürzen.

Erfindungsgemäß wird diese Aufgabe durch die Merkmale der Ansprüche 1 und 9 gelöst.

Beim erfindungsgemäßen Verfahren zur Handhabung von Schmelzproben in einem Stahlwerkslabor wird die Schmelzprobe in wenigstens einer ersten Behandlungseinrichtung bearbeitet und/oder analysiert und wird anschließend über wenigstens einen ersten Transportweg zu wenigstens einer zweiten Behandlungseinrichtung transportiert, wo die Schmelzprobe einer weiteren Bearbeitung und/oder Analyse unterzogen wird. Die Schmelzprobe wird dabei auf einem Probenträger eingespannt, sodass der Probenträger mit der eingespannten Schmelzprobe zwischen den Behandlungseinrichtungen transportiert und in den Behandlungseinrichtungen für die Bearbeitung und/oder Analyse positioniert wird.

Die Probe verbleibt somit bis zur letzten Behandlungseinrichtung auf dem Probenträger, sodass zwischendurch kein Umspannen und Vermessen und damit weniger Handhabung erforderlich sind. Da die Probenträger im Gegensatz zu den eingespannten Proben eine immer gleiche Form haben, ist zudem einen einfachere Handhabung beim Transfer zwischen einer Transporteinrichtung und der Behandlungseinrichtung gegeben.

Das erfindungsgemäße Stahlwerkslabor zur Handhabung von Schmelzproben sieht wenigstens eine erste und eine zweite Behandlungseinrichtung zur Bearbeitung und/oder Analyse der Schmelzproben sowie eine Transportvorrichtung zum Transport der Schmelzproben zwischen den Behandlungseinrichtungen vor. Weiterhin ist ein Probenträger mit einer ersten Spanneinrichtung zum Einspannen der Schmelzprobe vorgesehen, wobei der Probenträger mit einer eingespannten Probe für den Transport auf der Transportvorrichtung ausgebildet ist. Im Bereich der Behandlungsstationen ist ferner eine Transfereinheit zum Transferieren des Probenträgers mit der eingespannten Schmelzprobe in die Behandlungseinrichtung angeordnet.

Weitere Ausgestaltungen der Erfindung sind Gegenstand der Unteransprüche.

Gemäß einer Ausgestaltung der Erfindung wird der Probenträger mit der eingespannten Schmelzprobe für den Transport zwischen den wenigstens zwei Behandlungsstationen auf einer Transportvorrichtung platziert, die beispielsweise durch ein Förderband oder ein Schienensystem gebildet wird. Im Rahmen der Erfindung ist es aber auch denkbar, dass der Transport zwischen den Behandlungseinrichtungen mittels eines Roboters erfolgt. In einer weiteren Ausgestaltung kann der Probenträger mit der eingespannten Schmelzprobe zwischen der Transportvorrichtung und der wenigstens einen Behandlungseinrichtung transferiert werden, wobei auch eine Positionierung in einer gedrehten Stellung (180°), beispielsweise über Kopf, in der Behandlungsstation vorgesehen werden kann. Dies kann dann erforderlich sein, wenn die Analysen von unten erfolgen und die Schmelzprobe dabei über Kopf gehalten werden muss.

Bei bestimmten Behandlungen der Probe ist auch deren Lage und Höhe in der Behandlungseinrichtung von entscheidender Bedeutung. So kommt es beispielsweise bei der Funkenemissionsspektroskopie (OES) entscheidend auf die Lage der Probe für die Analyse der Schmelzprobe an. Dazu wird die Oberfläche der Schmelzprobe durch die Bildanalyse auf Fehlstellen analysiert, wobei durch die eindeutige Position der Schmelzprobe auf dem Probenträger eine Analyse im "sauberem" Bereich der Probenoberfläche durchgeführt werden kann. Die Höhe der Probe ist vor allem für die Fräsbearbeitung in einer Fräse entscheidend.

In einer bevorzugten Ausführungsform der Erfindung wird die auf dem Probenträger eingespannte Schmelzprobe daher vor dem Positionieren der Schmelzprobe in einer Messstation in ihrer Lage und Höhe bezüglich des Probenträgers vermessen, sodass diese Daten in den einzelnen Behandlungseinrichtungen je nach Bedarf genutzt werden können. So ist in den Behandlungseinrichtungen, in denen es auf eine exakte Ausrichtung ankommt, eine erneute Vermessung nicht mehr erforderlich. In einer weiteren Ausgestaltung können die Messdaten an wenigstens eine der Behandlungsstationen vor der Bearbeitung und/oder Analyse der Schmelzprobe übergeben werden.

In den Behandlungseinrichtungen können die Schmelzproben wahlweise einer Kühlung und/oder einer Oberflächenbearbeitung und/oder einer Röntgenfluoreszenzanalyse und/oder einer Analyse mittels Funkenemissionsspekrometrie unterzogen werden. Wird eine Oberflächenbearbeitung der Schmelzprobe durchgeführt, so kann die Behandlungseinrichtung mit einer Einspannvorrichtung versehen sein, die auf den in der Behandlungseinrichtung positionierten Probenträger zur Erhöhung der auf die Schmelzprobe wirkenden Spannkraft einwirkt. Eine solche zusätzliche Spannkraft ist insbesondere bei einer Fräse von Vorteil.

Die Schmelzproben und/oder der zugehörige Probenträger können zur Identifizierung der Schmelzproben gekennzeichnet werden, wobei der zugehörige Probenträger auch die Behandlungsinformationen (z. B. die Vermessungsdaten) der Schmelzprobe aufnimmt und an die Behandlungseinrichtungen übergibt.

Gemäß einer Ausgestaltung der Erfindung weist die erste Spanneinrichtung des Probenträgers Spannbacken auf, die zur Erhöhung des Reibwertes mit einer Mikropartikelbeschichtung versehen sind, um einen sicheren Halt der Probe zu gewährleisten. Es ist auch denkbar, dass der Probenträger mit einer zweiten Spanneinrichtung zum Einspannen einer weiteren Schmelzprobe ausgestattet ist, wodurch zwei Schmelzproben gemeinsam transportiert werden können. Dabei ist es besonders vorteilhaft, wenn eine Schmelzprobe auf der Oberseite und die andere Schmelzprobe auf der Unterseite des Probenträgers angeordnet sind, wodurch sich eine relativ kompakte Form ergibt. Außerdem kann auf diese Weise die Zeitdauer für die Probenpräparation, das Probenhandling und die chemische Analyse weiter verkürzt werden. Insgesamt wird für diese drei voll automatisch ablaufenden Prozessschritte, inklusive des Transports der Probe in das Labor, eine Zeitdauer von lediglich 2 bis 3 Minuten angestrebt.

Weitere Vorteile und Ausgestaltungen der Erfindung werden anhand der nachfolgenden Beschreibung und der Zeichnung näher erläutert.

In der Zeichnung zeigen
- Fig.1: ein Blockschaltbild eines erfindungsgemäßen Stahlwerklabors,
- Fig.2: eine dreidimensionale Darstellung des Probenträgers mit eingespannter Schmelzprobe,
- Fig. 3: eine schematische Darstellung einer Einspann- und Messeinrichtung zum Einspannen und Vermessen der Schmelzprobe,
- Fig. 4: eine schematische Darstellung des Probenträgers mit eingespannter Schmelzprobe auf der Transporteinrichtung,
- Fig. 5a und 5b: eine Draufsicht und eine dreidimensionale Darstellung der Einspannvorrichtung in einer Bearbeitungseinrichtung,
- Fig. 6a bis 6c: verschiedene Ansichten während des Transfers des Probenträgers von der Transporteinrichtung in die Behandlungseinrichtung und
- Fig. 7: eine dreidimensionale Darstellung eines Probenträgers für zwei Schmelzproben.

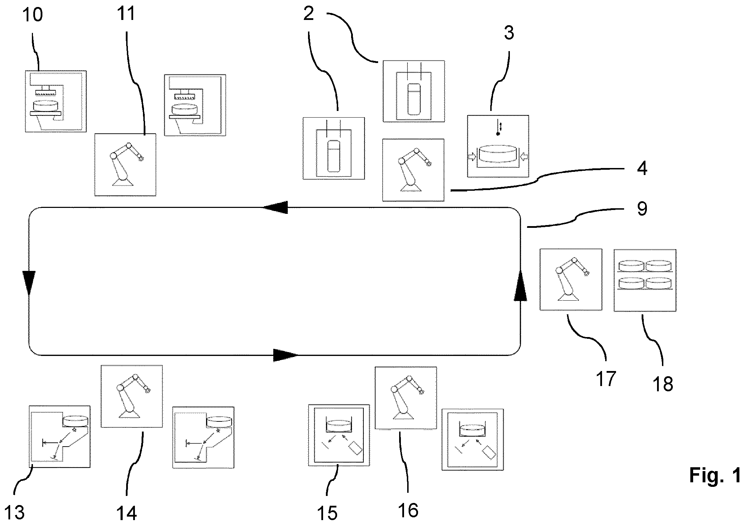

Fig. 1 zeigt ein Stahlwerkslabor zur Probenpräparation, zum Probenhandling und zur chemischen Analyse von Schmelzproben der Stahlherstellung. Die hier nicht näher dargestellte Probennahme erfolgt durch Eintauchen von speziellen Probennahmesonden in die Schmelze. Je nach Sonde werden zwischen Lollipop-Proben, Kegel- bzw. Kokillenproben, ovalen Proben und Laschenproben unterschieden, wobei Lollipop-Proben und ovale Proben bevorzugt werden, da sie nach Abtrennung des Stiels automatisch gehandhabt werden können.

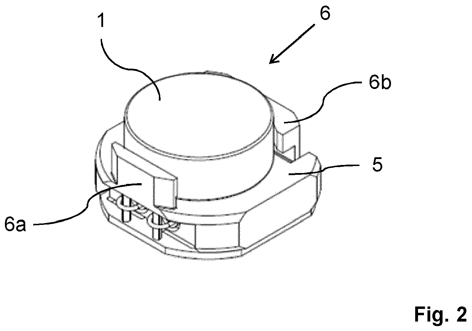

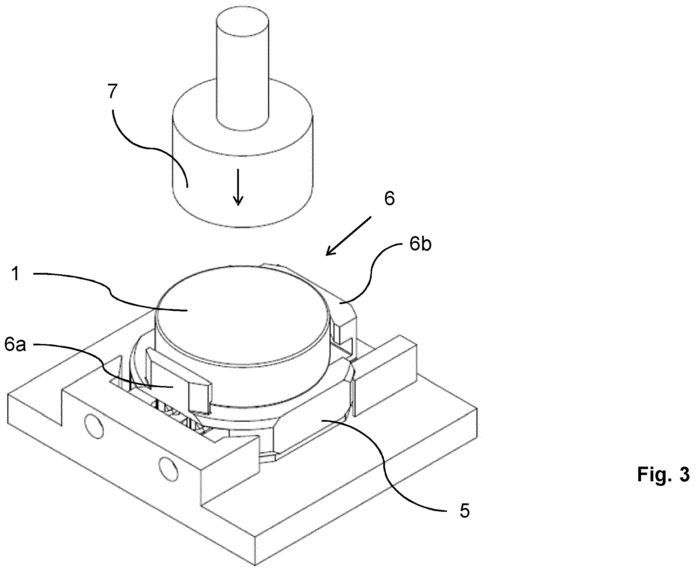

In der Nähe der Probennahmestelle werden die gezogenen Schmelzproben 1 einer Rohrpoststation übergeben und mit einer Rohrpostbüchse ins Stahlwerkslabor transportiert. Im Stahlwerkslabor gibt es wenigstens einen Rohrpostempfänger 2, der die Rohrpostbüchse automatisch öffnet und die Schmelzprobe 1 entnimmt. Die Schmelzprobe 1 wird einer Einspann- und Messstation 3 beispielsweise mit einem Roboter 4 übergeben (Fig. 3). Gemäß Fig. 2 wird die Schmelzprobe dort auf einem Probenträger 5 eingespannt. Der Probenträger 5 weist hierzu eine erste Spanneinrichtung 6 auf, die hier durch einen feststehenden Spannbacken 6a und einen federvorgespannten Spannbacken 6b gebildet wird. Die beiden Spannbacken 6a, 6b können zur Erhöhung der Haltekraft mit einer Mikropartikelbeschichtung versehen sein. Anschließend wird die am Probenträger 5 eingespannte Schmelzprobe 1 mit wenigstens einer Messeinrichtung 7 vermessen, wobei insbesondere die Lage und die Höhe der Schmelzprobe 1 bezüglich des Probenträgers 5 ermittelt wird. Die Messdaten werden entweder am Probenträger 5, beispielsweise mit einem RFID-System gespeichert oder direkt an die Behandlungseinrichtungen weitergeleitet. In jedem sind entweder die Schmelzproben und/oder die zugehörigen Probenträger zur Identifizierung der Schmelzprobe 1 in geeigneter Weise gekennzeichnet, sodass jede Schmelzprobe 1 an den jeweiligen Behandlungseinrichtungen eindeutig identifiziert werden kann.

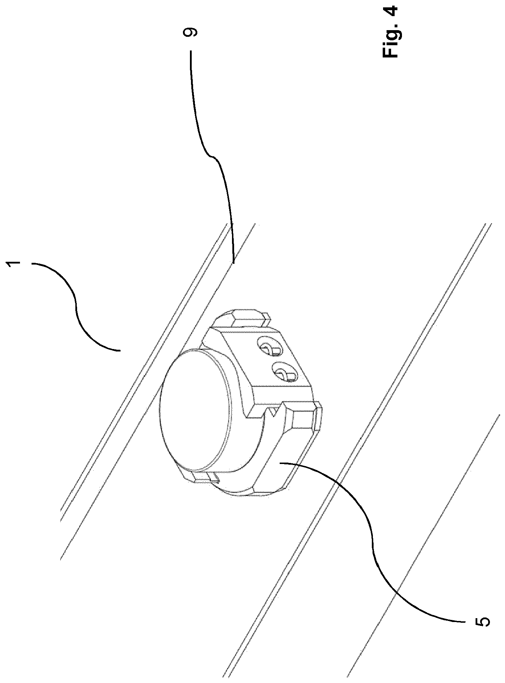

Der Probenträger 5 wird mit der eingespannten Schmelzprobe 1 mittels des Roboters 4 aus der Einspann- und Messstation 3 auf eine Transportvorrichtung 9 gesetzt, um den Probenträger 5 zu einer ersten Behandlungsstation 10 zu transportieren (Fig. 4), die hier als Fräse ausgebildet ist. Der Transfer von der Transporteinrichtung 9 in die ersten Behandlungsstation 10 erfolgt durch eine geeignete Transfereinrichtung, die beispielsweise durch einen zweiten Roboter 11 gebildet wird. Dabei wird der Probenträger 5 mit der eingespannten Schmelzprobe 1 in die Fräsmaschine transferiert.

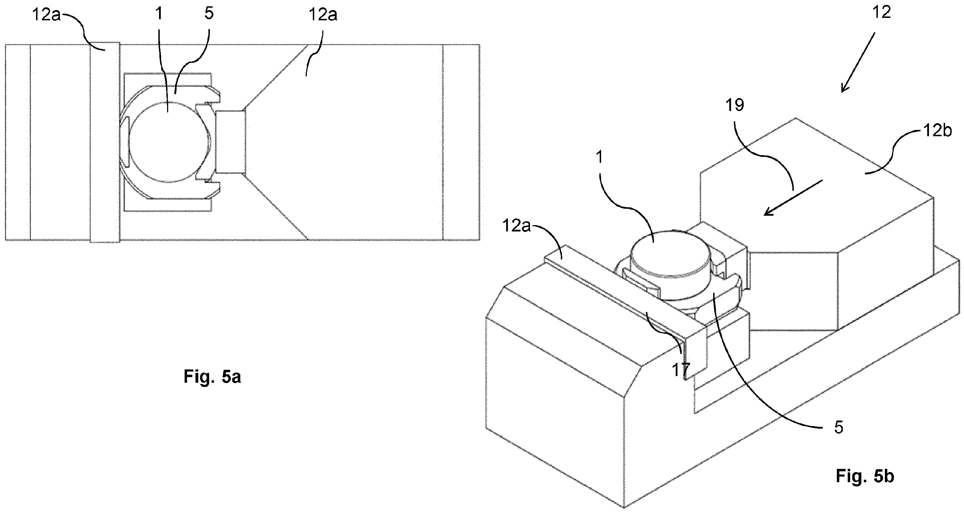

Die oberste Schicht der Schmelzprobe ist durch Oxidation gekennzeichnet und weist eine für die Schmelzprobe nicht repräsentative ca. 0,5 mm dicke Zunderschicht auf. Darunter schließt sich eine für eine repräsentative Analyse geeignete einige Millimeter dicke Schicht an. Die Zunderschicht auf der Schmelzprobe 1 muss daher ganzflächig entfernt werden. Hierzu hat sich der Einsatz einer Fräse etabliert. Da beim Fräsvorgang erhöhte Kräfte auf die Schmelzprobe 1 einwirken, weist die erste Behandlungsstation 10 (Fräse) eine zusätzliche Einspannvorrichtung auf, die bei in der ersten Behandlungseinrichtung 10 positioniertem Probenträger 5 mit der ersten Spanneinrichtung 6 des Probenträgers 5 zur Erhöhung der auf die Schmelzprobe 1 wirkenden Spannkraft in Wirkkontakt steht (Figuren 5a, 5b). Die Einspannvorrichtung 12 der ersten Behandlungseinrichtung 10 besteht aus einer feststehenden Backe 12a und einer beweglichen Backe 12b, wobei der Probenträger 5 derart in der Spannvorrichtung 12 platziert wird, dass der feststehende Spannbacken 6a des Probenträgers 5 mit der feststehenden Backe 12a und der federvorgespannte Spannbacken 6b mit der beweglichen Backe 12b in Wirkkontakt kommen. Durch die zusätzliche Kraft (Pfeil 19) der beweglichen Backe 12b kann die gesamte, auf die Schmelzprobe wirkende Spannkraft erhöht und dadurch auch beim Fräsen ein ausreichender Halt sichergestellt werden.

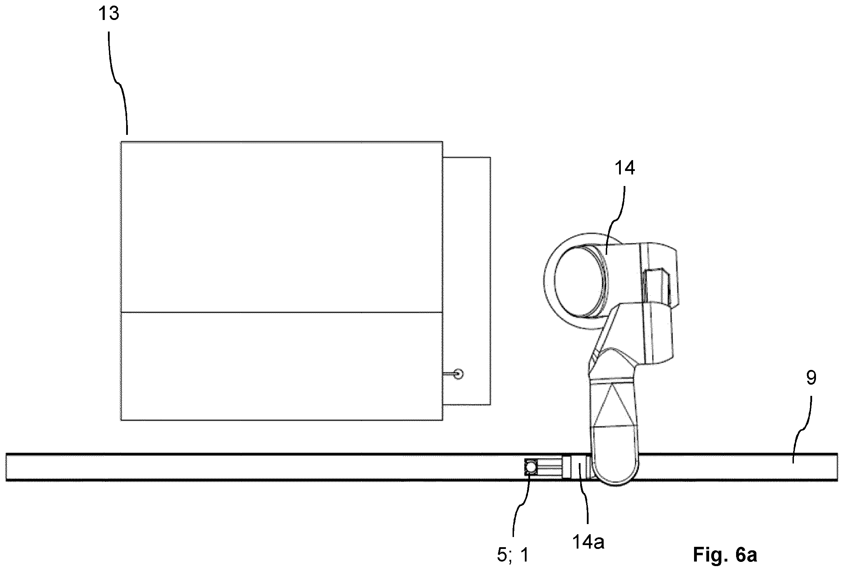

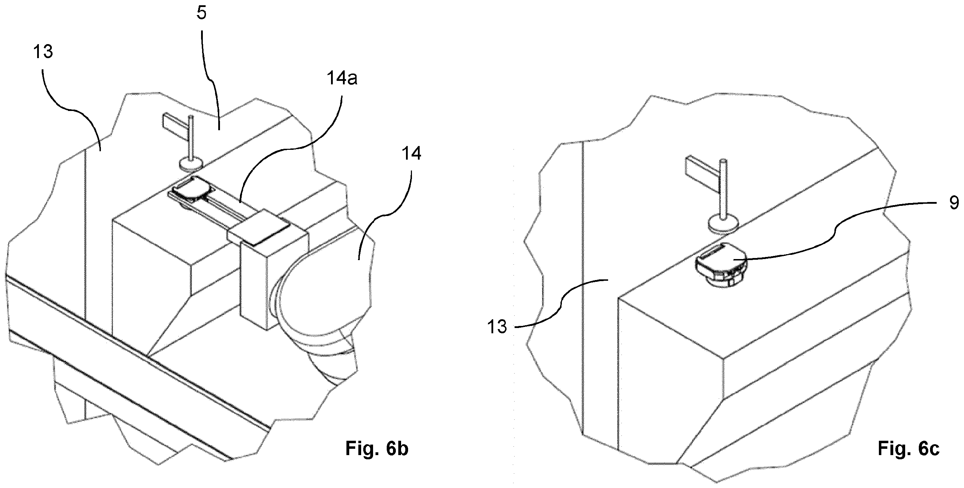

Nach den Fräsarbeiten wird der Probenträger 5 samt eingespannter Schmelzprobe 1 durch den zweiten Roboter 11 wieder auf die Transportvorrichtung 9 gesetzt, um die zweite Behandlungseinrichtung 13 anzusteuern. Hierbei handelt es sich beispielsweise um ein Gerät zur Funkenemissionsspektroskopie (OES), wodurch eine schnell chemische Analyse der Schmelzprobe 1 und eine Darstellung des Emissionsspektrums von chemischen Stoffen möglich ist. Die OES-Analyse ist wie die RFA-Analyse zur qualitativen und quantitativen Bestimmung der elementaren Zusammensetzung einer Probe bestimmt, wobei die Unterschiede in der Genauigkeit und der Analysedauer liegen. Der Transfer von der Transportvorrichtung 9 in die zweite Behandlungseinrichtung 13 ist in den Figuren 6a bis 6c dargestellt und erfolgt hier durch einen dritten Roboter 14 mit geeignetem Greifer 14a.

Als dritte Behandlungseinrichtung 15 ist ein Gerät zur Röntgenfluoreszenzanalyse (RFA) vorgesehen, die eine Methode zur qualitativen und quantitativen Bestimmung der elementaren Zusammensetzung einer Probe darstellt und den Vorteil hat, dass die Proben durch die Messung nicht zerstört und keine Aufschlüsse benötigt werden. Der Transfer des Probenträgers 5 mit der eingespannten Schmelzprobe 1 erfolgt hier über einen vierten Roboter 16.

Nachdem alle Analysen an der Schmelzprobe 1 durchgeführt wurden, gelangt diese mit Hilfe eines fünften Roboters in ein Probenlager, wo die Schmelzprobe aus dem Probenträger 5 herausgenommen wird, sodass der Probenträger für eine neu Schmelzprobe verwendet werden kann. Das Transportsystem ist daher auch zweckmäßigerweise als endlose Schleife ausgebildet, sodass der leere Probenträger als nächstes wieder zur Einspann- und Messstation 3 gelangt.

Das in Fig. 1 dargestellte Stahlwerkslabor ist lediglich als ein denkbares Ausführungsbeispiel zu sehen. Im Rahmen der Erfindung sind aber auch andere Ausgestaltungen denkbar. So können insbesondere in Abhängigkeit der Anzahl der zu analysierenden Schmelzproben die einzelnen Behandlungsstationen auch mehrfach vorhanden sein. Auch ist es denkbar, dass mehrere Behandlungsstationen im Wirkbereich einer einzelnen Transfereinrichtung (Roboter) angeordnet sind.

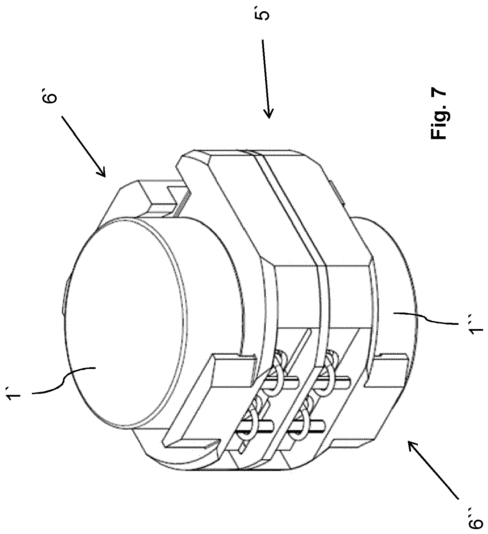

Fig. 7 zeigt schließlich noch ein zweites Ausführungsbeispiel für einen Probenträger 5', der so ausgebildet ist, dass zwei Schmelzproben 1', 1" aufgenommen werden können. Im dargestellten Ausführungsbeispiel wird der Probenträger 5' im Wesentlichen dadurch gebildet, dass zwei Probenträger gemäß Fig. 2 auf ihrer der eingespannten Schmelzprobe gegenüberliegenden Seite miteinander verbunden sind, sodass eine erste Schmelzprobe 1' oben und eine zweite Schmelzprobe 1" unten mit jeweils separaten Spanneinrichtungen 6', 6" eingespannt werden können. Auf diese Weise können zwei Schmelzproben gleichzeitig transportiert werden und auch die Behandlung in den Behandlungseinrichtungen kann schneller erfolgen, da die Probenträger 5' durch die Transfereinrichtung (Roboter) lediglich umgedreht werden muss.

## Patentansprüche

1. Verfahren zur Handhabung von Schmelzproben (1) in einem Stahlwerkslabor, wobei die Schmelzprobe (1) in wenigstens einer ersten Behandlungseinrichtung (10) bearbeitet und/oder analysiert wird und anschließend über wenigstens einen ersten Transportweg zu wenigstens einer zweiten Behandlungseinrichtung (13, 15) transportiert wird, wo die Schmelzprobe (1) einer weiteren Bearbeitung und/oder Analyse unterzogen wird,
**dadurch gekennzeichnet, dass** die Schmelzprobe (1) auf einem Probenträger (5) eingespannt wird und der Probenträger (5) mit der eingespannten Schmelzprobe (1) zwischen den Behandlungseinrichtungen (10, 13, 15) transportiert und in den Behandlungseinrichtungen (10, 13, 15) für die Bearbeitung und/oder Analyse positioniert wird.

2. Verfahren nach Anspruch 1, **dadurch gekennzeichnet, dass** der Probenträger mit der eingespannten Schmelzprobe (1) für den Transport zwischen den wenigstens zwei Behandlungseinrichtungen (10, 13, 15) auf einer Transportvorrichtung (9) platziert wird.

3. Verfahren nach Anspruch 1, **dadurch gekennzeichnet, dass** der Probenträger (5) mit der eingespannten Schmelzprobe (1) an jeder der Behandlungseinrichtungen (10, 13, 15) von der Transportvorrichtung (9) in die Behandlungseinrichtung (10, 13, 15) transferiert wird.

4. Verfahren nach Anspruch 3, **dadurch gekennzeichnet, dass** der Probenträger (5) mit der eingespannten Schmelzprobe (1) beim Transfer zwischen der Transportvorrichtung (9) und wenigstens einer der Behandlungseinrichtungen (10, 13, 15) in einer gedrehten Stellung in der Behandlungsstation (10, 13, 15) positioniert wird.

5. Verfahren nach Anspruch 1, **dadurch gekennzeichnet, dass** die auf dem Probenträger (5) eingespannte Schmelzprobe (1) vor dem Positionieren der Schmelzprobe (1) in einer der Behandlungseinrichtungen (10, 13, 15) in ihrer Lage und Höhe bezüglich des Probenträgers (5) vermessen wird und die Messdaten an wenigstens eine der Behandlungseinrichtungen (10, 13, 15) vor der Bearbeitung und/oder Analyse der Schmelzprobe (1) übergeben werden.

6. Verfahren nach Anspruch 1, **dadurch gekennzeichnet, dass** die Schmelzproben (1) in den wenigstens zwei Behandlungseinrichtungen (10, 13, 15) wahlweise einer Kühlung und/oder einer Oberflächenbearbeitung und/oder einer Röntgenfluoreszenzanalyse und/oder einer Analyse mittels Funkenemissionsspekrometrie unterzogen werden.

7. Verfahren nach Anspruch 1, **dadurch gekennzeichnet, dass** in wenigstens einer der beiden Behandlungseinrichtungen (10) eine Oberflächenbearbeitung der Schmelzprobe durchgeführt wird, wobei in der Behandlungseinrichtung (10) eine Einspannvorrichtung (12) vorgesehen ist, die auf den in der Behandlungseinrichtung (10) positionierten Probenträger (5) zur Erhöhung der auf die Schmelzprobe (1) wirkenden Spannkraft einwirkt.

8. Verfahren nach Anspruch 1, **dadurch gekennzeichnet, dass** die Schmelzproben (1) und/oder der zugehörige Probenträger (5) zur Identifizierung der Schmelzproben gekennzeichnet werden.

9. Verfahren nach Anspruch 8, **dadurch gekennzeichnet, dass** der zugehörige Probenträger (5) auch Behandlungsinformationen zu der Schmelzprobe (1) aufnimmt und an die Behandlungseinrichtungen (10, 13, 15) übergibt.

10. Stahlwerkslabor zur Handhabung von Schmelzproben (1) mit wenigstens einer ersten und einer zweiten Behandlungseinrichtung (10, 13, 15) zur Bearbeitung und/oder Analyse der Schmelzproben (1) sowie einer Transportvorrichtung (9) zum Transport der Schmelzproben (1) zwischen den Behandlungseinrichtungen (10, 13, 15),
**dadurch gekennzeichnet, dass** ein Probenträger (5) mit einer ersten Spanneinrichtung (6) zum Einspannen der Schmelzprobe (1) vorgesehen ist und der Probenträger (5) mit einer eingespannten Schmelzprobe (1) für den Transport auf der Transportvorrichtung (9) ausgebildet ist und im Bereich der Behandlungsstationen (10, 13, 15) eine Transfereinheit (11, 14, 15) zum Transferieren des Probenträgers (5) mit der eingespannten Schmelzprobe (1) in die Behandlungseinrichtung (10, 13, 15) vorgesehen ist.

11. Stahlwerkslabor nach Anspruch 10, **dadurch gekennzeichnet, dass** die wenigstens zwei Behandlungseinrichtungen (10, 13, 15) wahlweise durch eine Kühleinrichtung und/oder eine Oberflächenbearbeitungs-Einrichtung und/oder einer Röntgenfluoreszenzanalyse-Einrichtung und/oder ein Funkenemissionsspekrometer gebildet werden.

12. Stahlwerkslabor nach Anspruch 10, **dadurch gekennzeichnet, dass** wenigstens eine der beiden Behandlungseinrichtungen (10) durch eine Oberflächenbearbeitungs-Einrichtung gebildet wird, die eine Einspannvorrichtung (12) aufweist, die bei in der Behandlungseinrichtung (10) positioniertem Probenträger (5) mit der ersten Spanneinrichtung (6) des Probenträgers (5) zur Erhöhung der auf die Schmelzprobe (1) wirkenden Spannkraft in Wirkkontakt steht.

13. Stahlwerkslabor nach Anspruch 10, **dadurch gekennzeichnet, dass** die erste Spanneinrichtung (6) des Probenträgers (5) Spannbacken (6a, 6b) aufweist, die zur Erhöhung des Reibwertes mit einer Mikropartikelbeschichtung versehen sind.

14. Stahlwerkslabor nach Anspruch 10, **dadurch gekennzeichnet, dass** der Probenträger (5') mit einer zweiten Spanneinrichtung (6") zum Einspannen einer weiteren Schmelzprobe (1") ausgestattet ist.

## Claims

1. A method for handling melt samples (1) in a steelworks laboratory, wherein the melt sample (1) is processed and/or analyzed in at least one first treatment apparatus (10) and is subsequently transported via at least one first transport path to at least one second treatment apparatus (13, 15) where the melt sample (1) is subjected to further processing and/or analysis,
**characterized in that** the melt sample (1) is clamped on a sample carrier (5) and the sample carrier (5) is transported together with the clamped-in melt sample (1) between the treatment apparatuses (10, 13, 15) and positioned in the treatment apparatuses (10, 13, 15) for processing and/or analysis.

2. The method as claimed in claim 1, **characterized in that** the sample carrier together with the clamped-in melt sample (1) is placed on a transport apparatus (9) for transport between the at least two treatment apparatuses (10, 13, 15).

3. The method as claimed in claim 1, **characterized in that** the sample carrier (5) together with the clamped-in melt sample (1) is transferred from the transport apparatus (9) into the treatment apparatus (10, 13, 15) at each of the treatment apparatuses (10, 13, 15).

4. The method as claimed in claim 3, **characterized in that** the sample carrier (5) together with the clamped-in melt sample (1) is positioned in a rotated position in the treatment station (10, 13, 15) during transfer between the transport apparatus (9) and at least one of the treatment apparatuses (10, 13, 15).

5. The method as claimed in claim 1, **characterized in that** the melt sample (1) clamped on the sample carrier (5) is measured in terms of its position and height relative to the sample carrier (5) before positioning of the melt sample (1) in one of the treatment apparatuses (10, 13, 15) and the measurement data are transmitted to at least one of the treatment apparatuses (10, 13, 15) before processing and/or analysis of the melt sample (1).

6. The method as claimed in claim 1, **characterized in that** the melt samples (1) are optionally subjected to cooling and/or a surface treatment and/or X-ray fluorescence analysis and/or an analysis by means of spark emission spectrometry in the at least two treatment apparatuses (10, 13, 15).

7. The method as claimed in claim 1, **characterized in that** a surface treatment of the melt sample is carried out in at least one of the two treatment apparatuses (10), where a clamping device (12) which acts on the sample carrier (5) positioned in the treatment apparatus (10) in order to increase the clamping force acting on the melt sample (1) is provided in the treatment apparatus (10).

8. The method as claimed in claim 1, **characterized in that** the melt samples (1) and/or the associated sample carrier (5) are marked to identify the melt samples.

9. The method as claimed in claim 8, **characterized in that** the associated sample carrier (5) also records treatment information regarding the melt sample (1) and transmits it to the treatment apparatuses (10, 13, 15).

10. A steelworks laboratory for handling melt samples (1), comprising at least one first and a second treatment apparatus (10, 13, 15) for processing and/or analysis of the melt samples (1) and also a transport apparatus (9) for transporting the melt samples (1) between the treatment apparatuses (10, 13, 15),
**characterized in that** a sample carrier (5) having a first clamping device (6) for clamping in the melt sample (1) is provided and the sample carrier (5) together with a clamped-in melt sample (1) is configured for transport on the transport apparatus (9) and a transfer unit (11, 14, 15) for transferring the sample carrier (5) together with the clamped-in melt sample (1) into the treatment apparatus (10, 13, 15) is provided in the region of the treatment stations (10, 13, 15).

11. The steelworks laboratory as claimed in claim 10, **characterized in that** the at least two treatment apparatuses (10, 13, 15) are optionally formed by a cooling device and/or a surface treatment apparatus and/or an X-ray fluorescence analysis apparatus and/or a spark emission spectrometer.

12. The steelworks laboratory as claimed in claim 10, **characterized in that** at least one of the two treatment apparatuses (10) is formed by a surface treatment apparatus which has a clamping device (12) which is in operative contact with the first clamping device (6) of the sample carrier (5) when the sample carrier (5) is positioned in the treatment apparatus (10) in order to increase the clamping force acting on the melt sample (1).

13. The steelworks laboratory as claimed in claim 10, **characterized in that** the first clamping device (6) of the sample carrier (5) has clamping jaws (6a, 6b) which are provided with a microparticle coating to increase the coefficient of friction.

14. The steelworks laboratory as claimed in claim 10, **characterized in that** the sample carrier (5') is equipped with a second clamping device (6") for clamping-in a further melt sample (1").

## Revendications

1. Procédé pour manipuler des échantillons de matière fondue (1) dans un laboratoire sidérurgique, l'échantillon de matière fondue (1) étant traité et/ou analysé dans au moins un premier dispositif de traitement (10) et étant ensuite transporté par le biais d'au moins une première voie de transport vers au moins un deuxième dispositif de traitement (13, 15), où l'échantillon de matière fondue (1) est soumis à un traitement et/ou une analyse supplémentaire,
**caractérisé en ce que** l'échantillon de matière fondue (1) est serré sur un porte-échantillon (5) et le porte-échantillon (5) avec l'échantillon de matière fondue serré (1) est transporté entre les dispositifs de traitement (10, 13, 15) et positionné dans les dispositifs de traitement (10, 13, 15) pour le traitement et/ou l'analyse.

2. Procédé selon la revendication 1, **caractérisé en ce que** le porte-échantillon avec l'échantillon de matière fondue serré (1) est placé sur un dispositif de transport (9) pour le transport entre les au moins deux dispositifs de traitement (10, 13, 15).

3. Procédé selon la revendication 1, **caractérisé en ce que** le porte-échantillon (5) avec l'échantillon de matière fondue serré (1) est transféré au niveau de chacun des dispositifs de traitement (10, 13, 15) du dispositif de transport (9) dans le dispositif de traitement (10, 13, 15).

4. Procédé selon la revendication 3, **caractérisé en ce que** le porte-échantillon (5) avec l'échantillon de matière fondue serré (1) est positionné selon un orientement tourné dans la station de traitement (10, 13, 15) lors du transfert entre le dispositif de transport (9) et au moins l'un des dispositifs de traitement (10, 13, 15).

5. Procédé selon la revendication 1, **caractérisé en ce que** la position et la hauteur de l'échantillon de matière fondue (1) serré sur le porte-échantillon (5) par rapport au porte-échantillon (5) sont mesurées avant le positionnement de l'échantillon de matière fondue (1) dans l'un des dispositifs de traitement (10, 13, 15) et les données de mesure sont transmises à au moins l'un des dispositifs de traitement (10, 13, 15) avant le traitement et/ou l'analyse de l'échantillon de matière fondue (1).

6. Procédé selon la revendication 1, **caractérisé en ce que** les échantillons de matière fondue (1) sont soumis dans les au moins deux dispositifs de traitement (10, 13, 15), au choix, à un refroidissement et/ou à un traitement de surface et/ou à une analyse par fluorescence X et/ou à une analyse par spectrométrie d'émission optique.

7. Procédé selon la revendication 1, **caractérisé en ce que,** dans au moins l'un des deux dispositifs de traitement (10), un traitement de surface de l'échantillon de matière fondue est réalisé, un dispositif de serrage (12) étant prévu dans le dispositif de traitement (10), lequel agit sur le porte-échantillon (5) positionné dans le dispositif de traitement (10) pour augmenter la force de serrage agissant sur l'échantillon de matière fondue (1).

8. Procédé selon la revendication 1, **caractérisé en ce que** les échantillons de matière fondue (1) et/ou le porte-échantillon associé (5) sont marqués pour identifier les échantillons de matière fondue.

9. Procédé selon la revendication 8, **caractérisé en ce que** le porte-échantillon associé (5) recueille également des informations de traitement concernant l'échantillon de matière fondue (1) et les transmet aux dispositifs de traitement (10, 13, 15).

10. Laboratoire sidérurgique pour manipuler des échantillons de matière fondue (1), comprenant au moins un premier et un deuxième dispositif de traitement (10, 13, 15) pour traiter et/ou analyser les échantillons de matière fondue (1), ainsi qu'un dispositif de transport (9) pour transporter les échantillons de matière fondue (1) entre les dispositifs de traitement (10, 13, 15), **caractérisé en ce qu'**un porte-échantillon (5) comprenant un premier dispositif de serrage (6) est prévu pour serrer l'échantillon de matière fondue (1) et le porte-échantillon (5) avec un échantillon de matière fondue serré (1) est configuré pour le transport sur le dispositif de transport (9) et, dans la zone des stations de traitement (10, 13, 15), une unité de transfert (11, 14, 15) est prévue pour transférer le porte-échantillon (5) avec l'échantillon de matière fondue serré (1) dans le dispositif de traitement (10, 13, 15).

11. Laboratoire sidérurgique selon la revendication 10, **caractérisé en ce que** les au moins deux dispositifs de traitement (10, 13, 15) sont formés, au choix, par un dispositif de refroidissement et/ou un dispositif de traitement de surface et/ou un dispositif d'analyse par fluorescence X et/ou un spectromètre d'émission optique.

12. Laboratoire sidérurgique selon la revendication 10, **caractérisé en ce qu'**au moins l'un des deux dispositifs de traitement (10) est formé par un dispositif de traitement de surface qui présente un dispositif de serrage (12) qui, lorsque le porte-échantillon (5) est positionné dans le dispositif de traitement (10), est en contact actif avec le premier dispositif de serrage (6) du porte-échantillon (5) pour augmenter la force de serrage agissant sur l'échantillon de matière fondue (1).

13. Laboratoire sidérurgique selon la revendication 10, **caractérisé en ce que** le premier dispositif de serrage (6) du porte-échantillon (5) présente des mâchoires de serrage (6a, 6b) qui sont pourvues d'un revêtement de microparticules pour augmenter le coefficient de frottement.

14. Laboratoire sidérurgique selon la revendication 10, **caractérisé en ce que** le porte-échantillon (5') est équipé d'un deuxième dispositif de serrage (6") pour serrer un autre échantillon de matière fondue (1").
